# EUROPÄISCHE PATENTSCHRIFT

(11) **EP 1 367 392 B1**
(45) Veröffentlichungstag und Bekanntmachung des Hinweises auf die Patenterteilung: **17.08.2005**
(21) Anmeldenummer: 03450130.4
(22) Anmeldetag: 22.05.2003
(51) Int. Cl.: G01N 33/49, A61B 5/00

(54) **Verfahren und Vorrichtung zur Messung von Blutgasparametern**
Method and device for measuring blood gas parameters
Procédé et dispositif pour mesurer des paramètres des gaz sanguins

(30) Priorität: 31.05.2002 AT 8392002
(43) Veröffentlichungstag der Anmeldung: 03.12.2003
(73) Patentinhaber: F.HOFFMANN-LA ROCHE AG, 4070 Basel (CH)
(72) Erfinder: Rüther, Horst, Dr. Dipl.-Ing., 8047 Hart/Graz (AT); Huemer, Herfried, Dipl.-Ing, 8330 Feldbach (AT)
(74) Vertreter: Babeluk, Michael, Dipl.-Ing.

(56) Entgegenhaltungen:
- WO-A-94/19684
- US-A- 4 114 602
- US-A- 4 290 431
- US-A- 4 548 259
- US-A- 5 232 667

## Beschreibung

Die Erfindung betrifft ein Verfahren und eine Vorrichtung zur Messung von Blutgasparametern, vorzugsweise pH, pCO₂ und pO₂ in einer Blutprobe, wobei die Blutprobe eines Patienten zumindest einer Messzelle eines Analysengerätes zugeführt wird.

Bei den in der Literatur beschriebenen und am Markt erhältlichen Blutgas-Multianalysatoren werden die Messzellen durch Heizen auf Körpertemperatur, das heißt auf 37,0°C, thermostatisiert. Mit den genannten Analysatoren werden neben den Blutgasparametern pH, pCO₂ und pO₂ meist auch Elektrolyte, metabolische Parameter und Hb-Derivate gemessen. Die maximale Betriebstemperatur derartiger Analysatoren muss deshalb immer unterhalb der Messzellentemperatur liegen und beträgt bei den am Markt erhältlichen Geräten 31°C bis 33°C.

In der US 4,548,259 A wird in Zusammenhang mit einem chemischen Analysator zur fotometrischen Vermessung einer flüssigen Probe eine Messzelle beschrieben, welche zu deren Thermostatisierung auf gegenüberliegenden Seiten Thermoelemente, beispielsweise Peltier-Elemente aufweist, wobei an den Seitenwänden der Messzelle weiters Temperatursonden angeordnet sind, mit welchen die Zellentemperatur gemessen werden kann. Nach Eingabe einer Probe in die Messzelle kann diese - in Abhängigkeit vom Ausgangssignal der Temperatursonden - mit Hilfe der Peltier-Elemente entweder erwärmt oder gekühlt werden bis eine vorgegebene Arbeitstemperatur im Bereich von 37°C ± 0,2°C erreicht wird. Die beschriebene Vorrichtung eignet sich somit beispielsweise für die photometrische Vermessung von Blutproben, bei welchen eine konstante Messtemperatur eingehalten werden muss.

Weiters ist aus der DE 26 51 356 A1 eine Messvorrichtung für die Fotometrie flüssiger Messproben bekannt, bei welcher für die Thermostatisierung kleiner Messgefäße (und somit kleiner Messproben) ein Peltier-Element als Wärmequelle und Wärmesenke vorgeschlagen wird. Damit lässt sich rasch und mit hoher Präzision die Arbeitstemperatur (bevorzugt 30°C) der Messvorrichtung einstellen.

Schließlich ist aus der US 4, 717, 548 ein Blutgasanalysator bekannt, welcher in einem extrakorporalen Blutkreislauf, beispielsweise während chirurgischer Eingriffe am Herzen, Blutgasparameter wie pH, pCO₂ und pO₂ sowie die Temperatur bestimmt, und die einzelnen Signale in Echtzeit aufzeichnet. Die Signale dienen zur Parameterüberwachung, wobei beim Überschreiten eingestellter Werte Schalt- und Alarmfunktionen ausgelöst werden.

Wenn mit herkömmlichen Blutgas-Multianalysatoren Blutgasuntersuchungen bei Patienten durchgeführt werden, deren Körpertemperatur von der Normaltemperatur (37°C) abweicht, beispielsweise während spezieller medizinischer Eingriffe, bei welchen die Körpertemperatur künstlich abgesenkt wird, ist es üblich, die vom Messgerät erhaltenen Werte für pH, pCO₂ und pO₂ nach mathematischen Modellen zu korrigieren, um die medizinisch relevanten Werte bei der aktuellen Körpertemperatur des Patienten zu erhalten. Derartige Korrekturen sind jedoch sehr unpräzise, so dass Fehlkalkulationen bis in den Bereich von 50% auftreten können.

Aufgabe der Erfindung ist es, ein Verfahren zur Messung der Blutgasparameter, vorzugsweise pH, pCO₂ und pO₂, einer Blutprobe, sowie ein Analysengerät zur Durchführung des Verfahrens vorzuschlagen, mit welchem es möglich ist, präzise Werte für die Blutgasparameter auch in jenen Fällen zu erhalten, wo die Patiententemperatur von der Normaltemperatur abweicht.

Diese Aufgabe wird erfindungsgemäß dadurch gelöst, dass die Temperatur des Patienten oder der Blutprobe bei der Entnahme gemessen wird, dass die gemessene Temperatur an das Analysengerät übermittelt oder durch das Analysengerät erfasst wird, sowie dass die Messzelle des Analysengerätes zur Messung der Blutgasparameter durch Kühlen oder Heizen auf die gemessene Temperatur geregelt wird.

Der Vorteil des erfindungsgemäßen Verfahrens besteht damit darin, die Partialdrücke (pO₂, pCO₂) und den pH-Wert bei der aktuellen Temperatur des Patienten zu messen, wodurch unmittelbar die physiologische relevanten Werte erfasst werden und jede nachträgliche, fehlerbehaftete mathematische Korrektur der Messwerte entfallen kann. Auf der Basis dieser Messwerte kann von medizinischer Seite rasch reagiert und beispielsweise Notfallmaßnahmen eingeleitet werden.

Ein Analysengerät zur Durchführung des erfindungsgemäßen Verfahrens zeichnet sich durch eine Regel- und Steuereinrichtung aus, mit welcher die Temperatur der Messzelle in Abhängigkeit von einer bei der Entnahme der Blutprobe gemessenen Blut- oder Patiententemperatur im Bereich von 22°C bis 40°C regelbar ist.

Mit dem erfindungsgemäßen Verfahren ist eine stabile Temperierung der Messzelle auch bei Betriebs- bzw. Umgebungstemperaturen möglich, welche über der Messzellentemperatur liegen.

Von besonderem Vorteil ist es, wenn die Messzelle bereits vor der Eingabe der Blutprobe in das Analysengerät auf die gemessene Temperatur abgekühlt oder erwärmt wird und die Probeneingabe erst bei Erreichen dieser Temperatur freigegeben wird. Durch diese Maßnahme kann der Internist bzw. Anästhesist beim medizinischen Eingriff entlastet werden, da der Analysator automatisch signalisiert, wann eine Probeneingabe aus messtechnischer Sicht zulässig.

Im einfachsten Fall der Erfindung kann die Blut- oder Patiententemperatur auf herkömmliche Art mittels Thermometer oder Messsonde gemessen und händisch in das Analysengerät über eine Eingabeeinrichtung (Tastatur oder Touch-Screen) eingegeben werden. Die Temperaturmessung, deren Eingabe in den Analysator, die Probennahme und die Probeneingabe in den Analysator sollten dabei bevorzugt innerhalb weniger Minuten erfolgen.

Von besonderem Vorteil ist allerdings eine Automatisierung dahingehend, dass die Blut- oder Patiententemperatur von einer im oder am Patienten angebrachten Temperaturmesssonde gemessen und automatisch an das Analysengerät übertragen wird. In diesem Fall ist es besonders vorteilhaft, wenn die Temperatur der Messzelle synchron der aktuell gemessenen Temperatur nachgeführt wird. Zu diesem Zweck kann die Patiententemperatur an verschiedenen Körperstellen mittels Thermometer, Temperatursensor oder Infrarotmessezelle gemessen werden. Für eine präzise Temperaturmessung empfiehlt sich beispielsweise die Platzierung einer Messsonde im Analbereich zur Ermittlung der Basaltemperatur.

Von Vorteil ist es, dass Messsignal direkt in der Messsonde zu verstärken, eine A/D-Wandlung durchzuführen und über eine Distanz von wenigen Metern zur Schnittstelle des Analysators per IF- oder per RF-Signal störungsfrei zu übertragen. Weiters ist es möglich, eine Signalübertragung per Kabel vorzusehen.

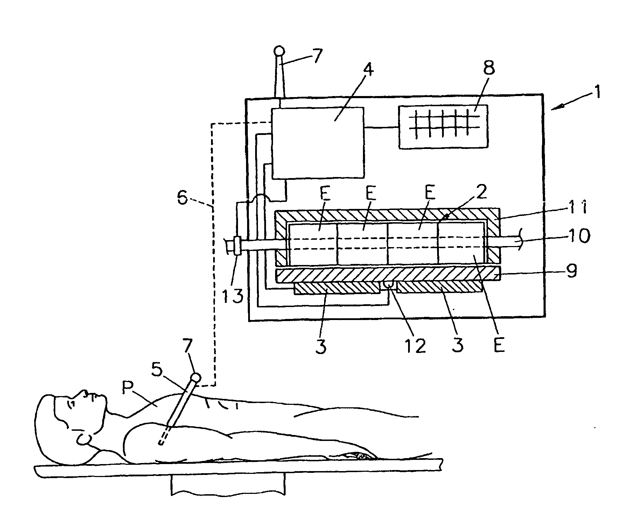

Die Erfindung wird im Folgenden anhand einer schematischen Darstellung in Fig. 1 näher erläutert.

Mit 1 ist ein Analysengerät zur Messung von Blutgasparametern, vorzugsweise pH, pCO₂ und pO₂, in einer Blutprobe bezeichnet, wobei das Analysengerät 1 zumindest eine thermostatisierbare Messzelle 2 mit elektrochemischen Messelektroden E, beispielsweise Durchflusssensoren, aufweist. Die Messzelle 2 bzw. die Messelektroden E können beispielsweise mit Hilfe von Peltier-Elementen 3 über ein thermisch gut leitendes Verteilerelement 9 gekühlt bzw. erhitzt werden - je nach Ausgangssignal einer Regel- und Steuereinrichtung 4, welche mit einer im oder am Patienten P anbringbaren Temperaturmesssonde 5 in Kontakt steht. Die jeweilige Ist-Temperatur, der von einer Probenkapillare 10 durchsetzten und mit einer thermischen Isolation 11 versehenen Messzelle 2, wird von einem Temperatursensor 12 erfasst und dessen Messsignal an die Regeleinrichtung 4 weitergeleitet.

Für die Datenübertragung zur Regel- und Steuereinrichtung 4 ist eine Kabelverbindung 6 (strichliert) oder eine Funkverbindung 7 vorgesehen. In Abhängigkeit der von der Temperaturmesssonde 5 gemessenen Patiententemperatur kann die Messzelle 2 im Bereich von 22°C bis 40°C geregelt werden, wobei die Temperatur der Messzelle 2 bevorzugt synchron der aktuell gemessenen Temperatur des Patienten P nachgeführt wird.

Die von der Messsonde 5 ermittelte Patiententemperatur kann auch abgelesen und mit Hilfe einer Eingabeeinrichtung 8, beispielsweise einer Tastatur, in das Analysengerät 1 eingegeben werden.

Weiters kann das Analysengerät 1 am Probeneingabeelement eine Sperreinrichtung 13 aufweisen, welche von der Regel- und Steuereinrichtung 4 ansteuerbar ist und das Probeneingabeelement erst bei Erreichen der gemessenen Blut- oder Patiententemperatur frei gibt.

## Patentansprüche

1. Verfahren zur Messung von Blutgasparametern, vorzugsweise pH, pCO₂ und pO₂ in einer Blutprobe, wobei die Blutprobe eines Patienten zumindest einer Messzelle eines Analysengerätes zugeführt wird, **dadurch gekennzeichnet, dass** die Temperatur des Patienten oder der Blutprobe bei der Entnahme gemessen wird, dass die gemessene Temperatur an das Analysengerät übermittelt oder durch das Analysengerät erfasst wird, sowie dass die Messzelle des Analysengerätes zur Messung der Blutgasparameter durch Kühlen oder Heizen auf die gemessene Temperatur geregelt wird.

2. Verfahren nach Anspruch 1, **dadurch gekennzeichnet, dass** die Messzelle bereits vor der Eingabe der Blutprobe in das Analysengerät auf die gemessene Temperatur abgekühlt oder erwärmt wird und die Probeneingabe erst bei Erreichen dieser Temperatur freigegeben wird.

3. Verfahren nach Anspruch 1 oder 2, **dadurch gekennzeichnet, dass** die Blut- oder Patiententemperatur von einer im oder am Patienten angebrachten Temperaturmesssonde gemessen und automatisch an das Analysengerät übertragen wird.

4. Verfahren nach einem der Ansprüche 1 bis 3, **dadurch gekennzeichnet, dass** die Temperatur der Messzelle synchron der aktuell gemessenen Temperatur nachgeführt wird.

5. Verfahren nach einem der Ansprüche 1 bis 4, **dadurch gekennzeichnet, dass** die Messzelle des Analysengerätes in Abhängigkeit der gemessenen Temperatur auf eine Temperatur im Bereich von 22°C bis 40°C geregelt wird.

6. Verfahren nach Anspruch 5, **dadurch gekennzeichnet, dass** die Übertragung der gemessenen Temperatur an das Analysengerät per Kabel, per IF- oder per RF-Signal erfolgt.

7. Analysengerät (1) zur Messung von Blutgasparametern, vorzugsweise pH, pCO₂ und pO₂ in einer Blutprobe mit zumindest einer thermostatisierbaren Messzelle (2), **dadurch gekennzeichnet dass** eine Regel- und Steuereinrichtung (4) vorgesehen ist, welche mit einer im oder am Patienten anbringbaren Temperaturmesssonde in Kontakt steht, so dass die Temperatur der Messzelle (2) in Abhängigkeit von einer bei der Entnahme der Blutprobe gemessenen Blut- oder Patiententemperatur im Bereich von 22°C bis 40°C regelbar ist.

8. Analysengerät nach Anspruch 7, **dadurch gekennzeichnet, dass** das Analysengerät (1) am Probeneingabeelement eine Sperreinrichtung (13) aufweist, welche von der Regel- und Steuereinrichtung (4) ansteuerbar ist und das Probeneingabeelement erst bei Erreichen der gemessenen Blut- oder Patiententemperatur frei gibt.

9. Analysengerät nach Anspruch 7 oder 8, **dadurch gekennzeichnet, dass** für die Datenübertragung von der Temperaturmesssonde (5) zur Regeleinrichtung (4) eine Kabel- oder Funkverbindung (6; 7) vorgesehen ist.

## Claims

1. A method for measuring blood gas parameters, preferably pH, pCO₂, and pO₂, in a blood sample, the blood sample of a patient being fed into at least one measuring cell of an analyzer, **characterized in that** the temperature of the patient or the blood sample is measured upon sample withdrawal, and that the measured temperature is transmitted to the analyzer or recorded by the analyzer, and that for measuring the blood gas parameters the measuring cell of the analyzer is adjusted to the measured temperature by cooling or heating.

2. A method according to claim 1, **characterized in that** the measuring cell is cooled or heated to the measured temperature before the blood sample is entered into the analyzer, and that sample entry is enabled only after this temperature is reached.

3. A method according to claim 1 or 2, **characterized in that** the blood or patient temperature is measured by a temperature probe placed in or on the patient and the temperature data are transmitted automatically to the analyzer.

4. A method according to any of claims 1 to 3, **characterized in that** the temperature of the measuring cell is continually adjusted synchronously with the actual temperature measured.

5. A method according to any of claims 1 to 4, **characterized in that** the temperature of the measuring cell of the analyzer is regulated in the range from 22°C to 40°C, depending on the temperature measured.

6. A method according to claim 5, **characterized in that** the transmission of the measured temperature to the analyzer is performed via cable, via IF signal, or via RF signal.

7. An analyzer (1) for measuring blood gas parameters, preferably pH, pCO₂ and pO₂, in a blood sample, with at least one thermostabilized measuring cell (2), **characterized in that** a regulating and control unit (4) is provided, which is connected to a temperature probe (5) placed in or on the patient, such that the temperature of the measuring cell (2) can be regulated in the range from 22°C to 40°C, depending on the temperature of the blood or patient measured upon sample withdrawal.

8. An analyzer according to claim 7, **characterized in that** the analyzer (1) is provided with a locking device (13) on the sample input element, which is controlled by the regulating and control device (4) and enables sample input only when the temperature is equal to the blood or patient temperature measured.

9. An analyzer according to claim 7 or 8, **characterized in that** a cable or wireless link (6; 7) is provided for data transmission from the temperature probe (5) to the regulating and control unit (4).

## Revendications

1. Procédé pour mesurer des paramètres des gaz du sang, de préférence le pH, la pCO₂ et la pO₂ dans un échantillon de sang, dans lequel l'échantillon de sang d'un patient est amené dans au moins une cellule de mesure d'un appareil d'analyse, **caractérisé en ce que** la température du patient ou de l'échantillon de sang est mesurée au moment du prélèvement, **en ce que** la température mesurée est transmise à l'appareil d'analyse ou enregistrée par l'appareil d'analyse, et **en ce que** la cellule de mesure de l'appareil d'analyse pour mesurer les paramètres des gaz du sang est réglée à la température mesurée au moyen d'un refroidissement ou d'un chauffage.

2. Procédé selon la revendication 1, **caractérisé en ce que** la cellule de mesure est refroidie ou chauffée à la température mesurée avant le chargement de l'échantillon de sang dans l'appareil d'analyse et **en ce que** le chargement de l'échantillon n'est autorisé que lorsque cette température a été atteinte.

3. Procédé selon la revendication 1 ou 2, **caractérisé en ce que** la température du sang ou du patient est mesurée par un capteur de température placé dans ou sur le patient et qu'elle est transmise automatiquement à l'appareil d'analyse.

4. Procédé selon l'une des revendications 1 à 3, **caractérisé en ce que** la température de la cellule de mesure est portée de manière synchrone à la température réelle mesurée.

5. Procédé selon l'une des revendications 1 à 4, **caractérisé en ce que** la cellule de mesure de l'appareil d'analyse est réglée en fonction de la température mesurée à une température allant de 22°C à 40°C.

6. Procédé selon la revendication 5, **caractérisé en ce que** la transmission de la température mesurée à l'appareil d'analyse se fait par câble, par signal IF ou RF.

7. Appareil d'analyse (1) pour mesurer des paramètres des gaz du sang, de préférence le pH, la pCO₂ et la pO₂, dans un échantillon de sang, comprenant au moins une cellule de mesure (2) à température réglable, **caractérisé en ce qu'**un dispositif de réglage ou de commande (4), qui est en contact avec un capteur de température pouvant être placé dans ou sur le patient, est prévu de sorte que la température de la cellule de mesure (2) est réglable dans une gamme de 22°C à 40°C en fonction d'une température du sang ou du patient mesurée au moment du prélèvement de l'échantillon sanguin.

8. Appareil d'analyse selon la revendication 7, **caractérisé en ce que** l'appareil d'analyse (1) présente un dispositif de blocage (13) au niveau de l'élément de chargement de l'échantillon sanguin, lequel peut être commandé par le dispositif de réglage et de commande (4) et débloque l'élément de chargement de l'échantillon sanguin uniquement lorsque la température du sang ou du patient mesurée a été atteinte.

9. Appareil d'analyse selon la revendication 7 ou 8, **caractérisé en ce qu'**une connexion câblée ou une connexion radio (6 ; 7) est prévue pour la transmission de données depuis le capteur de température (5) jusqu'au dispositif de réglage (4).
